# EUROPEAN PATENT APPLICATION

(11) **EP 3 995 024 A1**
(43) Date of publication of application: **11.05.2022**
(21) Application number: 19937126.1
(22) Date of filing: 05.07.2019
(51) Int. Cl.: A41D 13/12, A41D 31/26

(54) **ELECTROMAGNETIC WAVE PROTECTOR**

(71) Applicant: Medical-Aid Co., Ltd., Izumi-shi, Osaka 594-1144 (JP)
(72) Inventor: MATSUI Hideki, Izumi-shi, Osaka 594-1144 (JP)
(74) Representative: Zimmermann & Partner Patentanwälte mbB
(86) International application number: PCT/JP2019/026889
(87) International publication number: WO 2021/005664

(57) **Abstract**

The present invention addresses the problem of providing an electromagnetic wave protector having not only a superior electromagnetic wave shielding capability but also a significantly improved durability. An electromagnetic wave protector according to the present invention is attached to a region of a human body to shield the region from electromagnetic waves, and is provided with: a meshed electromagnetic wave shielding net composed of electrically conductive fibers and having a shape corresponding to an outer shape of the region; and a resin cover covering the electromagnetic wave shielding net.

## Description

### TECHNICAL FIELD

The present invention relates to an electromagnetic wave protector to be attached to an optional portion of a body in order to shield electromagnetic waves.

### BACKGROUND ART

An influence of electromagnetic waves generated from home electric products, office automation devices, engines, motors, welding machines, and the like on human bodies has become a problem. For example, a patient who uses an embedded-in-body medical device such as a cardiac pacemaker has a concern that the embedded-in-body medical device such as the cardiac pacemaker may malfunction due to the electromagnetic waves. Further, since strong electromagnetic waves are generated in a manufacturing site of an engine, a motor, or the like or a welding site, a worker who uses the embedded-in-body medical device such as the cardiac pacemaker is prohibited from working in these sites.

In view of such a background, the present applicant has proposed, in Patent Literature 1, a medical inner wear to be worn by a patient who uses an embedded-in-body medical device such as a cardiac pacemaker, an electromagnetic wave protective garment that prevents an endoscopic image from being interrupted due to an influence of electromagnetic waves during a capsule endoscope examination, and an electromagnetic wave protector that protects reproductive organs from electromagnetic waves of a microwave hyperthermia therapy device. The medical inner wear, the electromagnetic wave protective garment, and the electromagnetic wave protector are made of mesh-shaped electromagnetic wave shielding nets made of conductive fibers. However, these medical inner wears are intended to prevent development of metal allergy by silver-plating conductive fibers and to improve durability, but since these medical inner wears are in direct contact with a human body, washing is inevitable, silver plating is chlorinated or sulfurized due to sweat or the like, and the medical inner wears are also in contact with the human body or other clothes, and thus deterioration over time cannot be avoided.

Since a patient receives a strong RF pulse (electromagnetic waves of 64 MHz, 128

MHz, and 256 MHz are mainly used as electromagnetic waves radiated in a direction perpendicular to a static magnetic field) in an MRI examination, a patient who uses the cardiac pacemaker needs to electromagnetically shield a pacemaker main body and a lead wire that connects a pacemaker to a heart so as not to be influenced by the electromagnetic waves. Further, it is necessary to shield electromagnetic waves in order to prevent heat generation of a ring, an accessory, or a non-magnetic metal (a metal stent, a metal wire, an artificial joint, or the like) embedded in the body, and heat generation and discoloration of a metal component contained in a dye such as tattoos. Furthermore, it is necessary to perform electromagnetic shielding in order to prevent a burn caused by an induced current when a contact between a gantry, a coil, or a cable of MRI and a hand or a loop between a body and an arm is formed. Furthermore, since electromagnetic waves of the microwave hyperthermia therapy device may also influence the reproductive organs, it is necessary to shield a lower abdomen. In addition to the above prevention of the influence of the electromagnetic waves on the human body, it is possible to prevent aliasing artifacts (background reflection due to reflection) by shielding the RF pulse during MRI imaging.

Although the medical inner wear described in Patent Literature 1 can also be used as an electromagnetic wave protector that is to be attached to these portions during the MRI examination and is for shielding an RF pulse, as described above, there is a problem of deterioration over time, and handling such as washing is complicated.

### CITATION LIST

### PATENT LITERATURE

Patent Literature 1: Japanese Patent No. 4210326

### SUMMARY OF INVENTION

### TECHNICAL PROBLEM

The present invention has been made in view of the above circumstances, and an object thereof is to provide an electromagnetic wave protector that not only has excellent electromagnetic wave shielding ability, but also has significantly improved durability and handling such as washing.

### SOLUTION TO PROBLEM

In order to solve the above problems, the present invention provides the following electromagnetic wave protector.
(1) An electromagnetic wave protector to be attached to a portion in order to shield the portion of a body from an electromagnetic wave, the electromagnetic wave protector including:
   an electromagnetic wave shielding net having a mesh shape made of a conductive fiber and having a shape corresponding to an outer shape of the portion; and
   a resin cover surrounding the electromagnetic wave shielding net.
(2) The electromagnetic wave protector according to the above (1), further including: a closing material configured to close a gap between the portion and the resin cover when the electromagnetic wave protector is attached.
(3) The electromagnetic wave protector according to the above (1) or (2),
   in which the resin cover includes a first resin cover as one of an outer material and a lining, and a second resin cover as the other of the outer material and the lining,
   in which a plurality of sewn elements each including the electromagnetic wave shielding net and the first resin cover are sewn adjacent to each other,
   in which a resin cover sewn portion in which the first resin covers are sewn to each other and an electromagnetic wave shielding net sewn portion in which end edge portions of the adjacent electromagnetic wave shielding nets are sewn to each other are provided in an overlapping portion in which end edge portions of the adjacent first resin covers overlap each other, and
   in which the resin cover sewn portion is exposed as a joint portion, and the electromagnetic wave shielding net sewn portion is positioned in the first and second resin covers.
(4) The electromagnetic wave protector according to the above (3),
   in which end edge portions of the sewn element including the electromagnetic wave shielding net and the first resin cover are sewn to each other to form a tubular body,
   in which the tubular body includes the resin cover sewn portion in which the first resin covers are sewn to each other and the electromagnetic wave shielding net sewn portion in which the end edge portions of the electromagnetic wave shielding nets are sewn to each other in the overlapping portion in which the end edge portions of the first resin covers overlap each other, and
   in which the resin cover sewn portion is exposed as a joint portion, and the electromagnetic wave shielding net sewn portion is positioned in the first and second resin covers.
(5) The electromagnetic wave protector according to the above (1) or (2),
   in which the resin cover includes a first resin cover as one of an outer material and a lining, and a second resin cover as the other of the outer material and the lining,
   in which end edge portions of the sewn element including the electromagnetic wave shielding net and the first resin cover are sewn to each other to form a tubular body,
   in which the tubular body includes a resin cover sewn portion in which the first resin covers are sewn to each other and an electromagnetic wave shielding net sewn portion in which end edge portions of the electromagnetic wave shielding nets are sewn to each other in an overlapping portion in which end edge portions of the first resin covers overlap each other, and
   in which the resin cover sewn portion is exposed as a joint portion, and the electromagnetic wave shielding net sewn portion is positioned in the first and second resin covers.
(6) The electromagnetic wave protector according to any one of the above (3) to (5), in which in the electromagnetic wave shielding net sewn portion, the electromagnetic wave shielding nets are sewn to each other together with the first resin covers.
(7) The electromagnetic wave protector according to any one of the above (1) to (6), in which the electromagnetic wave protector has a shape in which no loop portion is formed on a surface perpendicular to an incident direction of the electromagnetic wave.
(8) The electromagnetic wave protector according to any one of the above (1) to (7), in which the electromagnetic wave protector is used for a patient of an MRI examination.
(9) The electromagnetic wave protector according to any one of the above (1) to (7), in which the electromagnetic wave protector is configured to be attached to a patient who uses a cardiac pacemaker.
(10) The electromagnetic wave protector according to any one of the above (1) to (7), in which the electromagnetic wave protector is configured to be attached to a patient of a capsule endoscope examination.
(11) The electromagnetic wave protector according to any one of the above (1) to (7), in which the electromagnetic wave protector is configured to be attached to a patient of a microwave hyperthermia therapy.

### ADVANTAGEOUS EFFECTS OF INVENTION

In an electromagnetic wave protector of the present invention, an electromagnetic wave shielding net in which conductive fibers are knitted in a mesh shape exhibits an excellent electromagnetic wave shielding effect. Further, since a surface is a resin cover, even when the electromagnetic wave protector is dirty, the dirt can be easily removed only by wiping with a tightly squeezed wet towel or an alcohol disinfection cloth, washing is not necessary, and since the surface is smooth, wear is also small. Therefore, the electromagnetic wave protector of the present invention can maintain an excellent electromagnetic wave shielding effect for a longer period of time than in the related art. Further, with a closing material, it is possible to more reliably shield electromagnetic waves by eliminating a gap between the electromagnetic wave protector and an attachment portion of a body as much as possible, and to apply the electromagnetic wave protector to patients having various body shapes and physical sizes.

### BRIEF DESCRIPTION OF DRAWINGS

Fig. 1 is a front view showing an electromagnetic wave shielding net used in an electromagnetic wave protector.
Fig. 2 is a cross-sectional view of the electromagnetic wave protector.
Fig. 3 is a schematic diagram showing a chest and abdomen electromagnetic wave protector as an example of the electromagnetic wave protector.
Fig. 4 is a schematic diagram showing a lower abdomen electromagnetic wave protector as another example of the electromagnetic wave protector.
Fig. 5 is a schematic diagram showing a hand, foot, and finger electromagnetic wave protector as another example of the electromagnetic wave protector.
Fig. 6 is schematic diagrams showing a neck electromagnetic wave protector as another example of the electromagnetic wave protector. (A) of Fig. 6 is a development view thereof, and (B) of Fig. 6 is a perspective view showing an attached state.
Fig. 7 is a schematic diagram showing a torso and abdomen electromagnetic wave protector as another example of the electromagnetic wave protector.
Fig. 8 is a diagram showing a shape in which no loop portion is formed in the electromagnetic wave protector, and is a modification of the chest and abdomen electromagnetic wave protector shown in Fig. 3.
Fig. 9A is a diagram for illustrating a sewing method of a sewn portion of two sewn elements, and is a diagram showing a step of sewing lining resin covers to each other.
Fig. 9B is a diagram for illustrating the sewing method of the sewn portion of the two sewn elements, in which (a) is a perspective view showing a step of sewing the sewn lining resin covers and electromagnetic wave shielding nets, and (b) is a schematic diagram seen from an A-A direction of (a).
Fig. 9C is a diagram for illustrating the sewing method of the sewn portion of the two sewn elements, and is a diagram showing a state where the lining resin covers are developed around the sewn portion of the lining resin covers, in which (b) is a schematic diagram seen from a B-B direction of (a).
Fig. 9D is a diagram for illustrating the sewing method of the sewn portion of the two sewn elements, in which (a) is a diagram showing a step of sewing outer material resin covers to the lining resin covers to which the electromagnetic wave shielding nets are sewn, and (b) is a schematic diagram seen from a C-C direction of (a).
Fig. 10A is a diagram for illustrating a sewing method of a sewn portion of a tubular sewn element, and is a diagram showing a step of sewing end edge portions of the outer material resin cover.
Fig. 10B is a diagram for illustrating the sewing method of the sewn portion of the tubular sewn element, and is a diagram showing a step of sewing the sewn outer material resin cover to the electromagnetic wave shielding nets.
Fig. 10C is a diagram for illustrating the sewing method of the sewn portion of the tubular sewn element, and is a diagram showing a step of sewing the lining resin cover to the outer material resin cover to which the electromagnetic wave shielding nets are sewn.

### DESCRIPTION OF EMBODIMENTS

Hereinafter, the present invention will be described in detail with reference to the drawings.

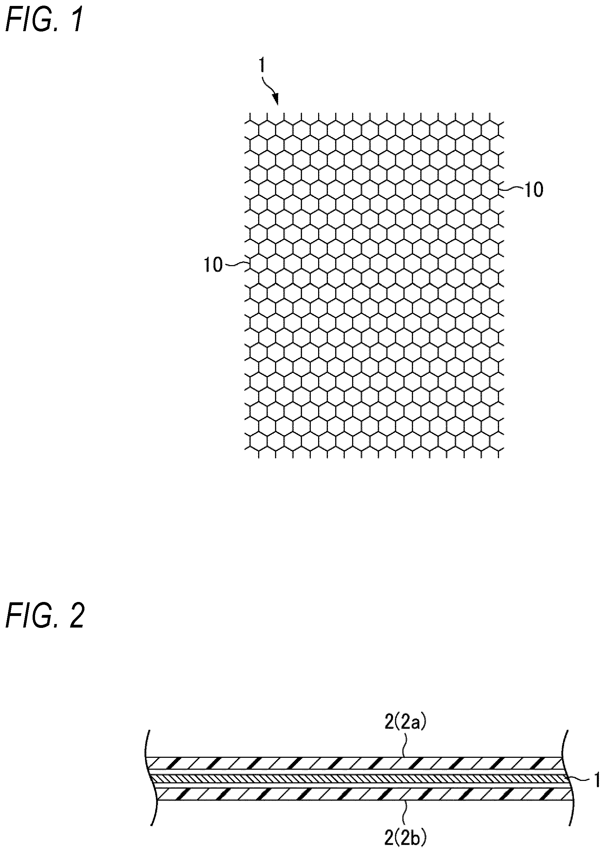

In an electromagnetic wave protector of the present invention, an electromagnetic wave shielding net is surrounded by a resin cover. Fig. 1 shows a front view of an electromagnetic wave shielding net 1. The electromagnetic wave shielding net 1 is formed by knitting conductive fibers 10 into a mesh shape, has plasticity and stretchability, and can be freely deformed. As the conductive fibers 10, in addition to metal fibers of silver, copper, nickel, or the like, conductive twisted yarns provided by twisting carbon fiber yarns or the like and synthetic resin fiber yarns of polyester or the like, twisted yarns provided by twisting two or more silver-plated single fibers, or the like can be used. Then, the conductive fibers 10 can be knitted with a general knitting machine such as a tricot knitting machine to form the electromagnetic wave shielding net 1. In the drawing, the conductive fibers 10 are knitted into a hexagonal shape, but may be knitted into another shape.

As the electromagnetic wave shielding net 1, an electromagnetic wave shielding net described in Patent Literature 1 can be referred to, and "MG net" manufactured by Medical Aid Co., Ltd. and sold by the present applicant can also be used.

Although one electromagnetic wave shielding net 1 may be used, an electromagnetic wave shielding effect is enhanced by using a plurality of electromagnetic wave shielding nets 1 in an overlapped manner.

Then, the electromagnetic wave shielding net 1 is formed into a shape conforming to an outer shape of a portion of a body to be attached, and is surrounded by a resin cover to serve as the electromagnetic wave protector of the present invention. Fig. 2 is a cross-sectional view of the electromagnetic wave shielding net 1, in which an outer material 2a on an atmosphere side and a lining 2b on a body side are made of resin sheets, the electromagnetic wave shielding net 1 is sandwiched between the outer material 2a and the lining 2b, and peripheral edges where the outer material 2a and the lining 2b are overlapped are sewn together. The resin sheets of the outer material 2a and the lining 2b serve as a resin cover 2. Further, the electromagnetic wave shielding net 1 is not joined to the outer material 2a and the lining 2b, and can be freely deformed.

Since the outer material 2a and the lining 2b, which serve as the resin cover 2, are both resin sheets and have water repellency and waterproofness, dirt can be removed only by wiping with a tightly squeezed wet towel or alcohol disinfecting cloth, and there is no need to wash the electromagnetic wave protector. Further, a surface is smooth, wear with other clothes is small, and durability is also excellent. Furthermore, coloring is also possible, and design properties can also be enhanced.

The resin is not limited, and any resin that is widely used for clothes can be used, and polyamide (nylon), polyester, or the like can be used. Since polyamide and polyester have a certain degree of heat resistance, hot water can also be used when wiping off dirt, and dirt removing performance is also excellent.

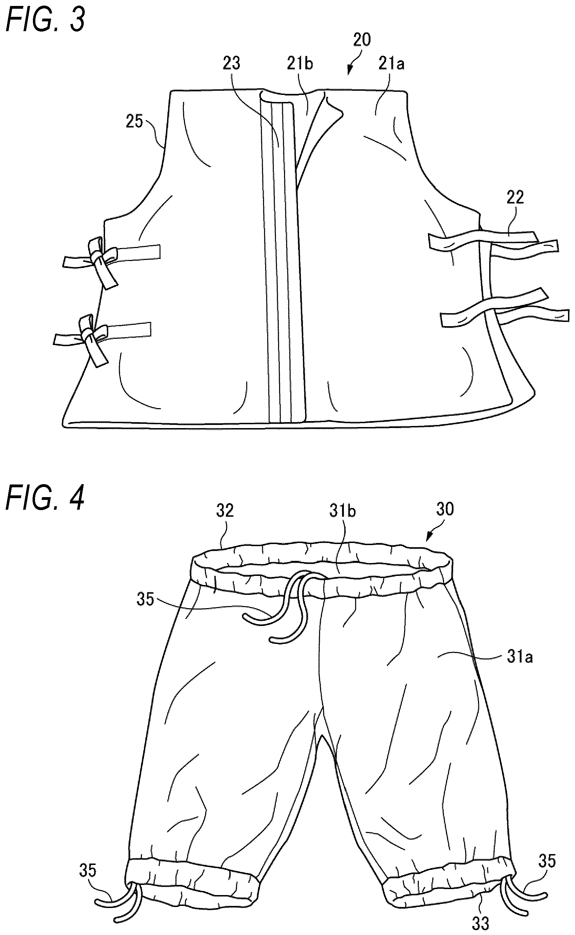

The electromagnetic wave protector of the present invention surrounds the electromagnetic wave shielding net 1 with the resin cover 2 as described above, and can take various forms depending on a portion of the body to be attached. Figs. 3 to 8 illustrate various electromagnetic wave protectors used during an MRI examination.

Fig. 3 is a schematic diagram showing an electromagnetic wave protector for protecting chest and abdomen (hereinafter, referred to as "chest and abdomen electromagnetic wave protector"), and the chest and abdomen electromagnetic wave protector 20 has a so-called "vest" shape as a whole. The electromagnetic wave shielding net is provided by knitting the conductive fibers into the mesh shape and then into the vest shape, and is, for example, a vest (not shown) made of "MG net" manufactured by Medical Aid Co., Ltd. Then, the vest of the electromagnetic wave shielding net is sandwiched between an outer material 21a and a lining 21b made of, for example, polyamide, and overlapped peripheral edges are sewn to provide the chest and abdomen electromagnetic wave protector 20. In the chest and abdomen electromagnetic wave protector 20, since the electromagnetic wave shielding net covers a chest, an abdomen, and a back of a patient during attachment, it is possible to protect a patient who uses a cardiac pacemaker during the MRI examination, to prevent heat generation of a non-magnetic metal (a metal stent, a metal wire, or the like) embedded in the chest, and to prevent heat generation or discoloration of a metal component contained in a dye such as tattoos. Further, by shielding an RF pulse of the chest during head MRI imaging, it is possible to prevent aliasing artifacts (background reflection due to reflection) of the chest.

Further, the chest and abdomen electromagnetic wave protector 20 can also be tied to a body portion with strings 22. Furthermore, a surface fastener 23 can also be opened and closed from the chest to the abdomen. With the strings 22 and the surface fastener 23, it is possible to eliminate gaps between the chest and abdomen electromagnetic wave protector 20 and the chest, the abdomen, and the back during attachment, and to apply the chest and abdomen electromagnetic wave protector 20 to patients having various body shapes and physical sizes.

Fig. 4 shows an electromagnetic wave protector for protecting a lower abdomen (hereinafter, referred to as "lower abdomen electromagnetic wave protector"), and the lower abdomen electromagnetic wave protector 30 has a semi-trousers shape as a whole. The electromagnetic wave shielding net is provided by knitting the conductive fibers into the mesh shape and then into the semi-trousers shape, and is, for example, semi-trousers (not shown) made of "MG net" manufactured by Medical Aid Co., Ltd. Then, the semi-trousers of the electromagnetic wave shielding net are sandwiched between an outer material 31a and a lining 31b made of, for example, polyamide, and overlapped peripheral edges are sewn to provide the lower abdomen electromagnetic wave protector 30. In the lower abdomen electromagnetic wave protector 30, the electromagnetic wave shielding net covers a lower abdomen of the patient during attachment, prevents heat generation of a stent or a coil in a pelvis and a non-magnetic metal implant embedded in a hip joint, prevents the heat generation and the discoloration of the metal component contained in the dye such as the tattoos, and protects reproductive organs.

A waist 32 and hems 33 have a bag shape, and the waist 32 and the hems 33 are tied by strings 35 during attachment, so that the waist 32 and the hems 33 can be narrowed to eliminate gaps between the lower abdomen electromagnetic wave protector 30 and the abdomen and thighs of the body. Further, opening dimensions of the waist 32 and the hems 33 can be adjusted by positions of knots of the strings 35, and the waist 32 and the hems 33 can be adapted to patients having different body shapes and physical sizes.

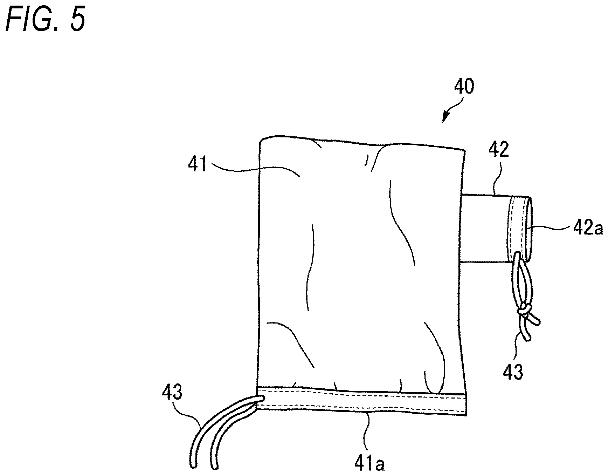

Fig. 5 shows an electromagnetic wave protector for protecting a hand, a foot, and a finger (hereinafter, referred to as "hand, foot, and finger electromagnetic wave protector"). The hand, foot, and finger electromagnetic wave protector 40 includes a bag-shaped main body portion 41 into which a hand or a foot is inserted, and a cylindrical branch portion 42 for introducing an emergency-contact air pump buzzer. Heat generation of a non-magnetic metal ring (particularly necessary when the ring cannot be removed) and the heat generation and the discoloration of the metal component contained in the dye such as the tattoos are prevented by shielding electromagnetic waves. The patient grips the emergency-contact air pump buzzer and notifies an external inspector when a poor physical condition occurs during the MRI examination, and an induced current may flow through a loop of a finger formed when the spherical air pump buzzer is gripped, and a burn may be caused. Therefore, when a hand or a finger is shielded, a structure is adopted in which shielding can be performed in a state where the emergency-contact buzzer is gripped.

The main body portion 41 is provided by preparing a part of a tubular body made of an electromagnetic wave shielding net, for example, the "MG net" manufactured by Medical Aid Co., Ltd with an opening corresponding to the branch portion 42, sandwiching the part of the tubular body between an outer material and a lining made of, for example, polyamide, and sewing the part of the tubular body in a bag shape along the overlapped peripheral edges. Further, the branch portion 42 is provided by sandwiching a tubular body made of the same net between an outer material and a lining made of, for example, polyamide, and sewing overlapped peripheral edges. Then, the branch portion 42 is attached to the opening of the main body portion 41, and the branch portion 42 and the main body portion 41 are sewn and integrated to form the hand, foot, and finger electromagnetic wave protector 40.

A hand and ankle insertion port 41a of the main body portion 41 and an insertion port 42a of the emergency-contact air pump buzzer of the branch portion 42 may be formed in a bag shape, and strings 43 may be passed through the respective openings. Then, when attached, the hand and ankle insertion port 41a and the insertion port 42a can be narrowed by the strings 43 to eliminate gaps between the hand, foot, and finger electromagnetic wave protector 40 and a wrist and the emergency-contact air pump buzzer.

Since the emergency-contact air pump buzzer may be gripped by any one of left and right hands, the shown hand, foot, and finger electromagnetic wave protector 40 may be for any one of the left and right hands, and the other only uses the main body portion 41.

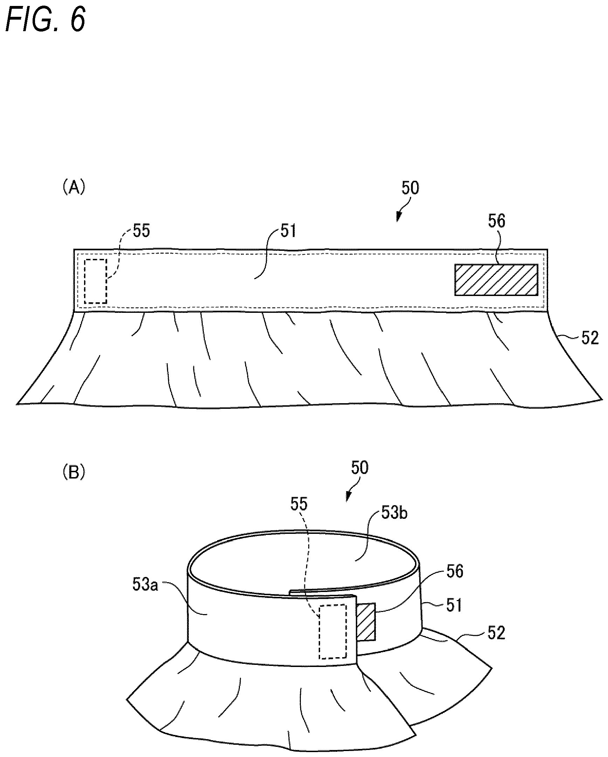

Fig. 6 shows an electromagnetic wave protector for protecting a neck (hereinafter, referred to as "neck electromagnetic wave protector"). (A) of Fig. 6 is a development view thereof, and (B) of Fig. 6 is a perspective view showing an attached state. As shown in (A) of Fig. 6, the neck electromagnetic wave protector 50 includes a band-shaped main body portion 51, and a skirt portion 52 sewn to one long side of the main body portion 51. Further, a surface fastener 55 is sewn to a lining 53b at one end of the main body portion 51 in a longitudinal direction, and a surface fastener 56 is sewn to an outer material 53a at the other end in the longitudinal direction. During attachment, as shown in (B) of Fig. 6, the main body portion 51 is rounded into a cylindrical shape, and is stopped by the surface fasteners 55 and 56. At the same time, the skirt portion 52 spreads in a circular shape, and covers an upper portion of the chest from a lower portion of the neck. It is possible to eliminate a gap in accordance with a thickness of the neck of the patient by positions where the surface fasteners 55 and 56 stop. The neck electromagnetic wave protector is mainly used in combination with the chest and abdomen electromagnetic wave protector, and it is possible to prevent aliasing artifacts (background reflection due to reflection) of the neck and the chest by preventing a tattoo around the neck where the electromagnetic waves cannot be shielded only by the chest and abdomen electromagnetic wave protector and heat generation of a non-magnetic metal embedded in the neck and by shielding RF pulses of the neck and the chest during the head MRI imaging.

The main body portion 51 is provided by sandwiching a band-shaped body made of an electromagnetic wave shielding net, for example, the "MG net" manufactured by Medical Aid Co., Ltd between the outer material 53a and the lining 53b made of, for example, polyamide, and sewing overlapped peripheral edges. The main body portion 51 is sewn with the surface fasteners 55 and 56. Further, the skirt portion 52 is provided by sandwiching a trapezoidal band body made of the same net between the outer material and the lining made of, for example, polyamide, and sewing overlapped peripheral edges.

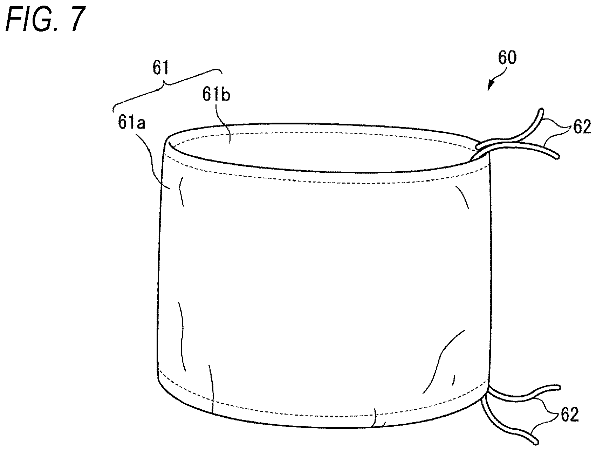

Fig. 7 shows an electromagnetic wave protector for protecting a torso and the abdomen (hereinafter, referred to as "torso and abdomen electromagnetic wave protector"), which is just in a form of "abdominal wrapping". The torso and abdomen electromagnetic wave protector 60 includes a tubular main body portion 61 that covers the torso and the abdomen. The main body portion 61 is provided by preparing a tubular body made of an electromagnetic wave shielding net, for example, the "MG net" manufactured by Medical Aid Co., Ltd, sandwiching the tubular body between an outer material 61a and a lining 61b made of, for example, polyamide, and sewing the tubular body into a tubular shape along overlapped peripheral edges. Further, a periphery of an upper end (a chest side) and a periphery of a lower end (a lower abdomen side) of the main body portion 61 may be formed in a bag shape, and strings 62 may be passed through the respective peripheries. Then, when attached, the periphery of the upper end (the chest side) and the periphery of the lower end (the lower abdomen side) can also be narrowed by the strings 62 to eliminate gaps between the torso and abdomen electromagnetic wave protector 60 and the torso and the abdomen.

In addition, although not shown in the drawings, the electromagnetic wave protector can also be formed in a single band-shaped body, and can also be used by being wound around a portion of the body, for example, an arm or a leg, which is desired to shield an RF pulse, to prevent heat generation of an artificial joint of a non-magnetic metal implanted in an elbow or a knee and the heat generation or the discoloration of the metal component contained in the dye such as the tattoos. Further, when a right foot is desired to be enhanced in contrast, aliasing artifacts of a left foot can be prevented by winding the electromagnetic wave protector around the left foot, and when a shoulder is desired to be enhanced in contrast, aliasing artifacts of the arm can be prevented by winding the electromagnetic protector below the elbow.

In this way, the electromagnetic wave protector of the present invention is easy to attach, can eliminate a gap between the electromagnetic wave protector and an attachment portion by the strings and the surface fastener to enhance a shielding effect, and can also be applied to patients having various body shapes and physical sizes. Further, since the outer material and the lining are made of resin, even when the outer material and the lining are dirty, it is only necessary to wipe off the dirt, and washing is not necessary, and since the surface is smooth, wear is small even when the surface rubs against an examination garment, and durability is also high. Furthermore, sterilization can be performed only by wiping with an alcohol disinfection cloth, which is also excellent in terms of hygiene and can also be reused.

Incidentally, in an MRI apparatus, since electromagnetic waves are radiated from a direction substantially perpendicular to each portion of a human body, the electromagnetic waves are also radiated to the electromagnetic wave protector in a direction substantially perpendicular to a surface of the electromagnetic wave protector. Therefore, for example, in the vest-shaped chest and abdomen electromagnetic wave protector 20 shown in Fig. 3, a side surface of the torso is also radiated by the electromagnetic waves substantially perpendicularly during attachment, and an arm hole denoted by a reference numeral 25 may be an opening that forms a loop portion on a surface perpendicular to an incident direction of the electromagnetic waves. Then, in the loop portion, an eddy current is generated and a temperature becomes high.

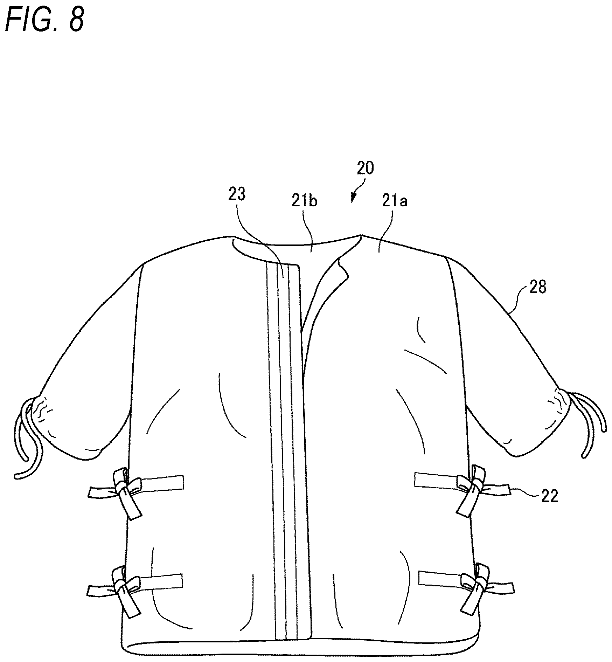

Therefore, as shown in Fig. 8, a shape in which sleeves 28 are attached is formed. A surface of the sleeve 28 is substantially perpendicular to the incident direction of the electromagnetic waves radiated from a left-right direction of a paper surface, and does not form the loop portion on the surface perpendicular to the incident direction of the electromagnetic waves.

Since surfaces of the lower abdomen electromagnetic wave protector 30, the hand, foot, and finger electromagnetic wave protector 40, the neck electromagnetic wave protector 50, and the torso and abdomen electromagnetic wave protector 60 shown above are all substantially perpendicular to the incident direction of the electromagnetic waves, no loop portion is formed on a surface perpendicular to the incident direction of the electromagnetic waves during attachment.

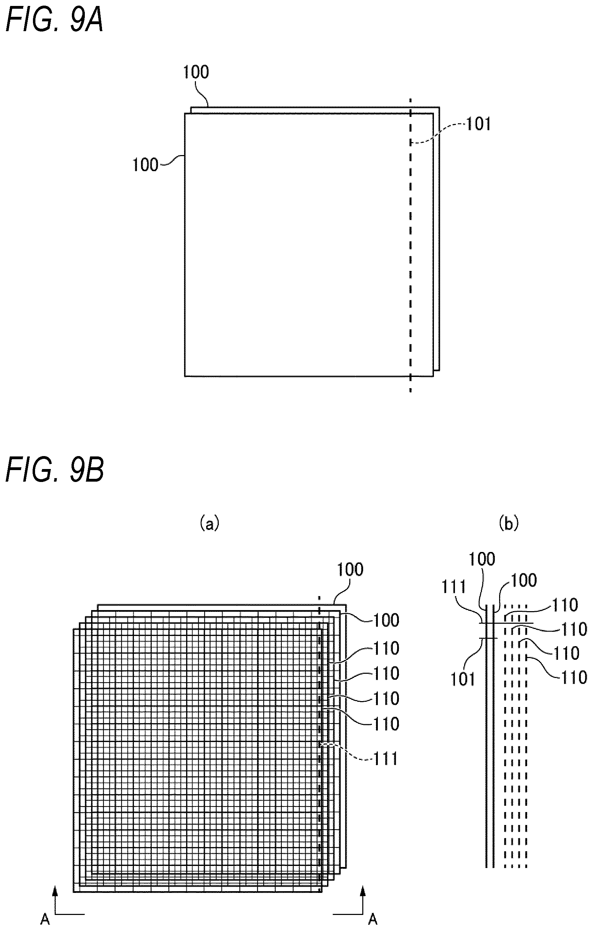

Seams of the electromagnetic wave protector in sewing are processed such that the electromagnetic wave shielding nets are electrically connected to each other. Specifically, sewing is performed as shown in Figs. 9A to 9D.

First, as shown in Fig. 9A, two resin covers 100 (hereinafter, referred to as "first resin covers"), which are one of the outer material and the lining, are sewn on one side in a state where the two resin covers 100 are overlapped with each other such that surfaces are in contact with each other. This sewn portion is denoted by reference numeral 101, and is referred to as a "resin cover sewn portion 101". The resin cover sewn portion 101 is positioned, for example, at an end edge portion 1 cm away from end edges of the first resin covers 100.

Next, as shown in (a) in Fig. 9B, electromagnetic wave shielding nets 110 are overlapped on an inside surface of one of the overlapped first resin covers 100, and are sewn together with the overlapped first resin cover 100 in parallel with the resin cover sewn portion 101 and at a position on an outer side of the resin cover sewn portion 101, for example, at a position of an end edge portion 5 mm away from the end edges of the first resin covers 100. The sewn portion is denoted by reference numeral 111, and is referred to as an "electromagnetic wave shielding net sewn portion". Further, (b) in Fig. 9B is a schematic diagram when (a) in Fig. 9A is viewed from an A-A direction.

In Fig. 9B, the number of the electromagnetic wave shielding nets 110 is two, but the number of the electromagnetic wave shielding nets 110 may be three or more, or may be one. Further, when there are two or more electromagnetic wave shielding nets 110, the electromagnetic wave shielding nets are overlapped and sewn together to be integrated.

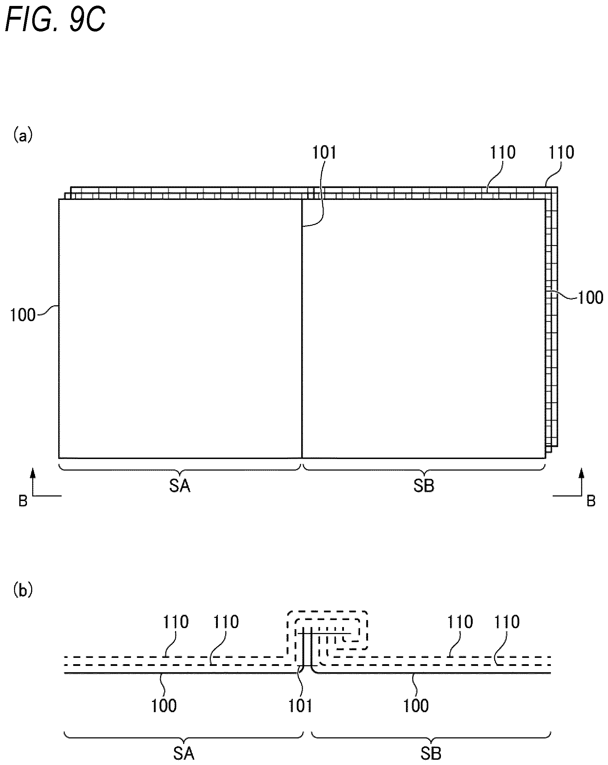

Fig. 9C shows a state where the first resin covers 100, to which the electromagnetic wave shielding nets 110 shown in Fig. 9B are sewn, are developed in the left-right direction of the paper surface around the resin cover sewn portion 101. Accordingly, a plurality of sewn elements SA and SB including the electromagnetic wave shielding nets 110 and the first resin covers 100 are in a state of being sewn adjacent to each other. Then, the resin cover sewn portion 101 where the first resin covers 100 are sewn together and the electromagnetic wave shielding net sewn portion 111 where end edge portions of the adjacent electromagnetic wave shielding nets 110 are sewn together are provided in an overlapping portion where the end edge portions of the adjacent first resin covers 100 overlap each other.

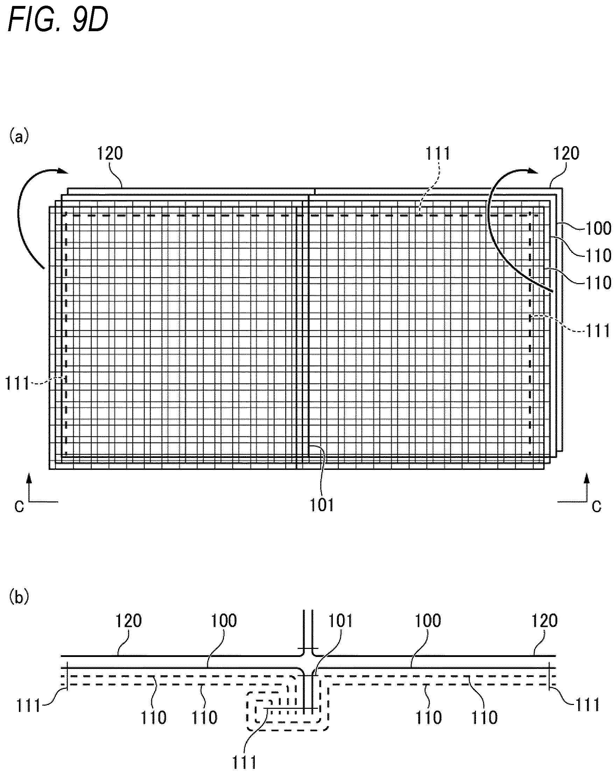

In the developed state, as shown in Fig. 9D, after the electromagnetic wave shielding nets 110 are sewn to three sides of the first resin covers 100, the other resin covers (hereinafter, referred to as "second resin covers") 120 that are the outer materials or the linings are sewn to surfaces of the first resin covers 100 on a side opposite to the electromagnetic wave shielding nets 110 in a state where surfaces face each other.

Then, as indicated by arrows in the same drawing, the first resin covers 100 to which the electromagnetic wave shielding nets 110 are sewn are turned over to a second resin cover 120 side, and the second resin covers 120 are sewn to the first resin covers 100. Accordingly, a three-layer structure in which the electromagnetic wave shielding nets 110 are sandwiched between the first resin covers 100 and the second resin covers 120 is provided. In the three-layer structure, the second resin covers 120 serve as an outer material, and the first resin covers 100 serve as a lining. Further, the resin cover sewn portion 101 is exposed as a joint portion, and the electromagnetic wave shielding net sewn portions 111 are positioned in the first and second resin covers 100 and 120.

Accordingly, since the electromagnetic wave shielding nets are electrically connected to each other at seams in the sewing, it is possible to prevent an increase in resistance at the seams, and to prevent a temperature from becoming high even when the induced current flows.

In the present embodiment, since the electromagnetic wave shielding nets 110 of the plurality of sewn elements SA and SB are sewn together with the overlapped first resin covers 100, the electromagnetic wave shielding nets 110 and the resin covers 100 and 120 do not need to be sewn together in advance, and the electromagnetic wave shielding net 110 and the resin covers 100 and 120 can be easily manufactured.

In the present invention, it is sufficient that the electromagnetic wave shielding nets 110 of the plurality of sewn elements SA and SB are electrically connected to each other at the seams in the sewing, and a sewing method other than the above embodiment may be used. For example, the plurality of sewn elements SA and SB in which the end edge portions of the electromagnetic wave shielding nets 110 are provided so as to extend from the end edges of the first resin covers 100 may be prepared, the surfaces of the first resin covers 100 may be overlapped with each other so as to be in contact with each other, the end edge portions of the first resin covers 100 may be sewn to each other, the end edge portions of the electromagnetic wave shielding nets 110 may be sewn to each other, and the sewn elements SA and SB may be developed in the left-right direction of the paper surface around the joint portion of the resin cover sewn portion 101, and may be sewn in the same manner as described above.

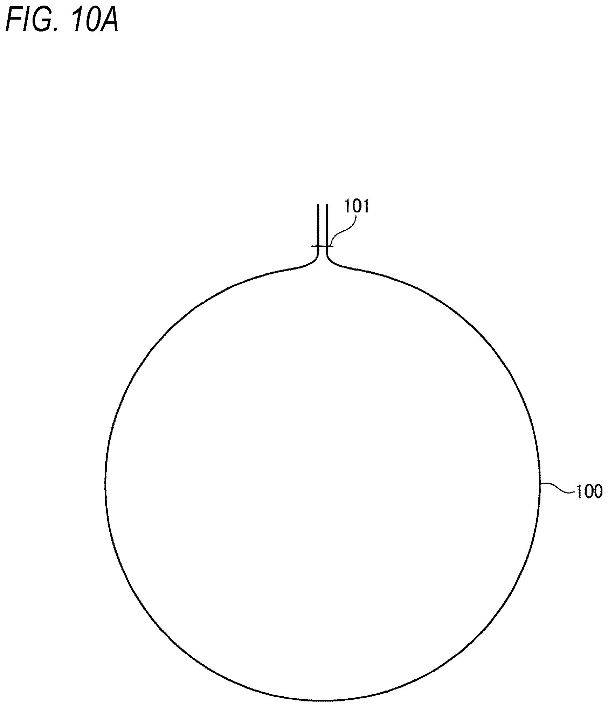

As shown in Figs. 10A to 10C, a portion such as the sleeve 28 is formed in a tubular shape by, for example, sewing end edge portions of two facing sides of one rectangular first resin cover 100, which is one of the outer material and the lining (the outer material in the drawing). Therefore, it is necessary to sew the electromagnetic wave shielding nets 110 such that the electromagnetic wave shielding nets 110 are electrically connected to each other also at seams of the sleeve 28.

First, as shown in Fig. 10A, in a state where the first resin cover 100 is curved such that the end edge portions of the outside surface of the first resin cover 100 are in contact with each other, the end edge portions of the two sides are sewn to each other to form the resin cover sewn portion 101.

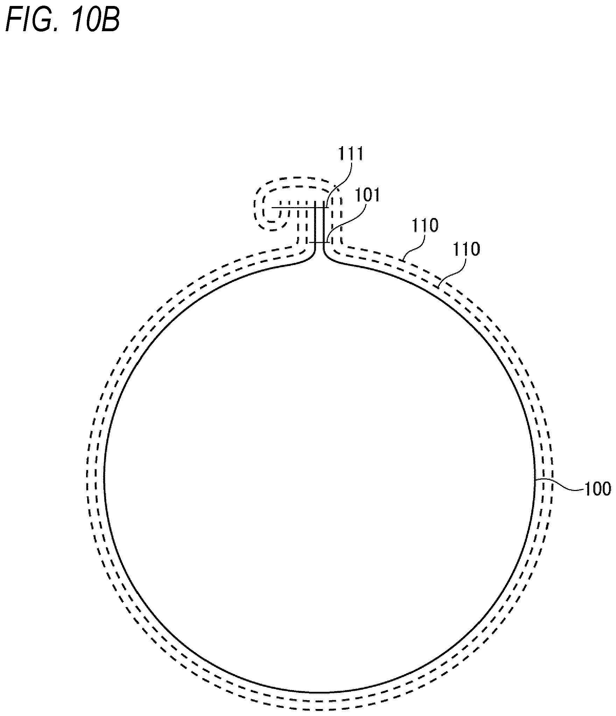

Next, as shown in Fig. 10B, the electromagnetic wave shielding nets 110 are overlapped around the inside surface of the first resin cover 100, and the end edge portions of the electromagnetic wave shielding nets 110 are arranged so as to overlap each other on one outer surface side of the resin cover sewn portion 101. Then, for example, at a position of 5 mm away from the end edge of the first resin cover 100, the end edge portions of the electromagnetic wave shielding nets 110 are sewn together with the overlapped first resin cover 100 to form the electromagnetic wave shielding net sewn portion 111. Accordingly, the end edge portions of the sewn element including the electromagnetic wave shielding nets 110 and the first resin cover 100 are sewn to each other to form a tubular body.

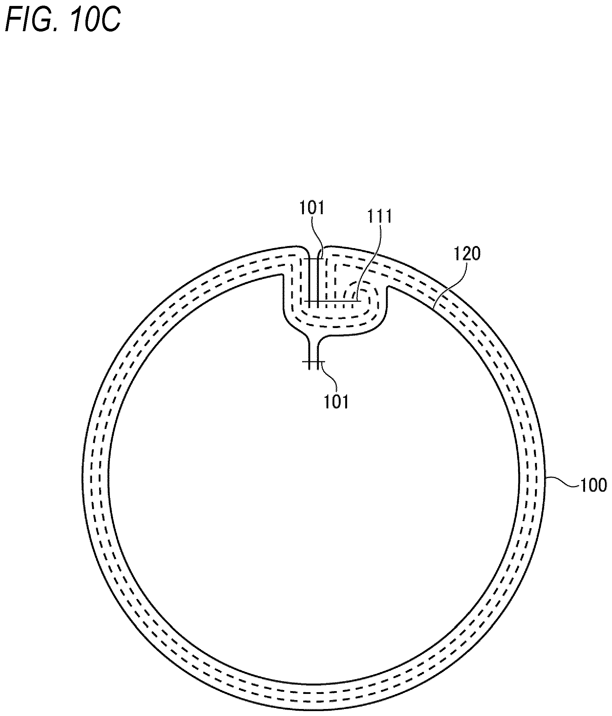

Then, as shown in Fig. 10C, outside and inside of the sewn element are turned over such that the outside surface of the first resin cover 100 becomes an outer peripheral surface. Further, the second resin cover 120 is formed in a tubular shape by sewing end edge portions of two facing sides of one rectangular second resin cover 120, which is the other of the outer material and the lining (the lining in the drawing). Thereafter, the first resin cover 100 and the second resin cover 120 are sewn together at end portions in a longitudinal direction to form the sleeve 28, and a three-layer structure in which the electromagnetic wave shielding nets 110 are sandwiched between the first resin cover 100 and the second resin cover 120 is provided. Further, the resin cover sewn portions 101 of the first and second resin covers 100 and 120 serve as joint portions and are exposed on outside and inside, respectively, and the electromagnetic wave shielding net sewn portion 111 is positioned in the first and second resin covers 100 and 120.

Accordingly, since the electromagnetic wave shielding nets are electrically connected to each other also at seams of the tubular portion such as the sleeve 28, it is possible to prevent an increase in resistance at the seams, and to prevent a temperature from becoming high even when the induced current flows.

The aspect in which the electromagnetic wave protector is attached during the MRI examination has been described above, but particularly, since the chest and abdomen electromagnetic wave protector 20 can shield an embedded medical device such as the cardiac pacemaker or a capsule endoscope from the RF pulse, the chest and abdomen electromagnetic wave protector 20 can also be used at times other than the time of the MRI examination. Since the electromagnetic waves cannot be seen, it is generally unclear where the electromagnetic waves are generated, but by attaching the chest and abdomen electromagnetic wave protector 20, a patient in which the embedded medical device such as the cardiac pacemaker is embedded or a patient who performs a capsule endoscope examination can be active regardless of a facility, a building, or a place.

### INDUSTRIAL APPLICABILITY

An electromagnetic wave protector of the present invention is useful as an examination garment during an MRI examination or a capsule endoscope examination, and is also useful for wearing during work of a patient in which an embedded medical device such as a cardiac pacemaker is embedded.

### REFERENCE SIGNS LIST

1: electromagnetic wave shielding net
2: resin cover
10: conductive fiber
20: chest and abdomen electromagnetic wave protector
30: lower abdomen electromagnetic wave protector
40: hand, foot, and finger electromagnetic wave protector
50: neck electromagnetic wave protector
60: torso and abdomen electromagnetic wave protector

## Claims

1. An electromagnetic wave protector to be attached to a portion of a body in order to shield the portion from an electromagnetic wave, the electromagnetic wave protector comprising:
an electromagnetic wave shielding net having a mesh shape made of a conductive fiber and having a shape corresponding to an outer shape of the portion; and
a resin cover surrounding the electromagnetic wave shielding net.

2. The electromagnetic wave protector according to claim 1, further comprising:
a closing material configured to close a gap between the portion and the resin cover when the electromagnetic wave protector is attached.

3. The electromagnetic wave protector according to claim 1 or 2, wherein
the resin cover includes a first resin cover as one of an outer material and a lining, and a second resin cover as the other of the outer material and the lining,
a plurality of sewn elements each including the electromagnetic wave shielding net and the first resin cover are sewn adjacent to each other,
a resin cover sewn portion in which the first resin covers are sewn to each other and an electromagnetic wave shielding net sewn portion in which end edge portions of the adjacent electromagnetic wave shielding nets are sewn to each other are provided in an overlapping portion in which end edge portions of the adjacent first resin covers overlap each other, and
the resin cover sewn portion is exposed as a joint portion, and the electromagnetic wave shielding net sewn portion is positioned in the first and second resin covers.

4. The electromagnetic wave protector according to claim 3, wherein
end edge portions of the sewn element including the electromagnetic wave shielding net and the first resin cover are sewn to each other to form a tubular body,
the tubular body includes the resin cover sewn portion in which the first resin covers are sewn to each other and the electromagnetic wave shielding net sewn portion in which the end edge portions of the electromagnetic wave shielding nets are sewn to each other in the overlapping portion in which the end edge portions of the first resin covers overlap each other, and the resin cover sewn portion is exposed as a joint portion, and the electromagnetic wave shielding net sewn portion is positioned in the first and second resin covers.

5. The electromagnetic wave protector according to claim 1 or 2, wherein
the resin cover includes a first resin cover as one of an outer material and a lining, and a second resin cover as the other of the outer material and the lining,
end edge portions of the sewn element including the electromagnetic wave shielding net and the first resin cover are sewn to each other to form a tubular body,
the tubular body includes a resin cover sewn portion in which the first resin covers are sewn to each other and an electromagnetic wave shielding net sewn portion in which end edge portions of the electromagnetic wave shielding nets are sewn to each other in an overlapping portion in which end edge portions of the first resin covers overlap each other, and
the resin cover sewn portion is exposed as a joint portion, and the electromagnetic wave shielding net sewn portion is positioned in the first and second resin covers.

6. The electromagnetic wave protector according to any one of claims 3 to 5, wherein in the electromagnetic wave shielding net sewn portion, the electromagnetic wave shielding nets are sewn to each other together with the first resin covers.

7. The electromagnetic wave protector according to any one of claims 1 to 6, wherein the electromagnetic wave protector has a shape in which no loop portion is formed on a surface perpendicular to an incident direction of the electromagnetic wave.

8. The electromagnetic wave protector according to any one of claims 1 to 7, wherein the electromagnetic wave protector is configured to be attached to a patient of an MRI examination.

9. The electromagnetic wave protector according to any one of claims 1 to 7, wherein the electromagnetic wave protector is configured to be attached to a patient who uses a cardiac pacemaker.

10. The electromagnetic wave protector according to any one of claims 1 to 7, wherein the electromagnetic wave protector is configured to be attached to a patient of a capsule endoscope examination.

11. The electromagnetic wave protector according to any one of claims 1 to 7, wherein the electromagnetic wave protector is configured to be attached to a patient of a microwave hyperthermia therapy.
